(19) Europäisches Patentamt
European Patent Office
Office européen des brevets

(11) **EP 2 769 666 A1**

(12) **EUROPEAN PATENT APPLICATION**
published in accordance with Art. 153(4) EPC

(43) Date of publication:
**27.08.2014 Bulletin 2014/35**

(51) Int Cl.:
***A61B 3/12*** *(2006.01)*

(21) Application number: **12840927.3**

(22) Date of filing: **18.10.2012**

(86) International application number:
**PCT/ES2012/070726**

(87) International publication number:
**WO 2013/057352 (25.04.2013 Gazette 2013/17)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.10.2011 ES 201131683**

(71) Applicants:
• **Asociación Industrial de Óptica, Color e Imagen - AIDO**
  **46980 Paterna (ES)**
• **Universitat de Valéncia**
  **46010 Valencia (ES)**

(72) Inventors:
• **MARTÍNEZ CORRAL, Manuel**
  **E-46010 Valencia (ES)**
• **PONS MARTÍ, Amparo**
  **E-46010 Valencia (ES)**
• **SAAVEDRA TORTOSA, Genaro**
  **E-46010 Valencia (ES)**
• **NAVARRO FRUCTUOSO, Héctor**
  **E-46010 Valencia (ES)**
• **MARTÍNEZ CUENCA, Raúl**
  **E-46010 Valencia (ES)**
• **TOLOSA RUIZ, Ángel**
  **E-46980 Paterna (ES)**
• **ALCÓN, Natividad**
  **E-46980 Paterna (ES)**

(74) Representative: **Carvajal y Urquijo, Isabel et al Clarke, Modet & Co.**
**Suero de Quiñones, 34-36**
**28002 Madrid (ES)**

(54) **MULTI-VIEW FUNDUS CAMERA**

(57)    The invention relates to a fundus camera that incorporates an integral imaging system for capturing an integral photograph of the fundus. This integral photograph enables projecting a three-dimensional image and generating topographical maps of the fundus. The fundus camera can function plenoptically and thus zoom onto a two-dimensional image generated from the integral photograph. The proposed equipment consists of an optical system for illuminating the fundus and an optical system forming the integral image capture system. This capture system includes an ophthalmoscopic lens, a microlens array and a sensor, and it allows recording, in a single shot, multiple views of the fundus. To improve the resolution of the integral photograph, the capture system has a device that allows displacing the microlens array by a few microns.

Fig. 1

**Description**

Field of the Art

The present invention is comprised in the field of optics and ophthalmology. It specifically relates to a fundus camera that captures an integral photograph of the fundus. Based on this integral photograph, it is possible to project three-dimensional images of the fundus, generate topographical maps of the fundus and perform optical sectioning on the image that is obtained.

Background

Fundus is a term used to refer to the posterior pole of the eyeball. This part primarily comprises the retina, the macula and the optic nerve, and the fundus is usually examined to detect eye diseases, also being possible to detect diseases not directly associated with the eye, such as diabetes or hypertension.

The most common devices for non-invasive examination of the retinal fundus are the ophthalmoscope and the fundus camera. These devices are very useful for a first diagnosis and for monitoring retinal pathologies. To improve the performances of said equipment, the proposed fundus camera is capable of generating three-dimensional images (3D) of the posterior pole. The main difference with respect to conventional ophthalmoscopes and fundus cameras, including stereoscopic ophthalmoscopes and fundus cameras, is based on the fact that the proposed camera records a large number of views, with horizontal and vertical parallax, that allow the actual reconstruction of the 3D image of the retinal fundus.

To project an integral image captured with the camera prepared for such purpose, another paper related to the present invention is the SPOC, smart pseudoscopic-to-orthoscopic conversion, algorithm proposed by H. Navarro, R. Martínez-Cuenca, G. Saavedra, M. Martínez-Corral, and B. Javidi in "3D integral imaging display by smart pseudoscopic-to-orthoscopic conversion," Opt. Express 25, 25573-25583 (2010), which allows adapting basic images of the original integral photograph that is captured to the characteristics necessary for projecting them from an integral imaging monitor or display and a correct 3D viewing.

Following is a review of other devices existing for obtaining images of the fundus.

The fundus camera, such as for example the one described in United States patent US 7,798,642 B2, incorporates an illumination system for illuminating the posterior pole of the eye, a low-power microscope and a camera coupled to it, which allows it to obtain photographs of the fundus. This device provides planar images without any possibility of making topographical reconstructions of the retinal fundus or 3D projections.

US 7,290,880 B1 describes a stereoscopic fundus camera. These cameras allow capturing two photographs of the posterior pole of the eye with a different horizontal view. These stereoscopic images are useful for calculating retinal topography, and also for being projected on stereoscopic monitors. However, as there are only two views, topographical reconstructions lack optical segmenting, i.e., it is not possible to select different planes in depth of scene in image reconstruction. In addition, and given that stereoscopic observation is very sensitive to effects inherent to the vergence-accommodation conflict, the projection of this stereoscopic image from a monitor gives rise to enormous visual fatigue when observed for prolonged periods.

The confocal scanning ophthalmoscope, based on the concept of the confocal microscopy, was proposed by Webb et al. in their article "Confocal scanning laser ophthalmoscope", Appl. Opt. 26, 1492-1499 in 1987. Not widely used due to its high cost, this apparatus is based on projecting a laser point focused by the eye optic on the retina and subsequently detecting the rays reflected by the retina after going through a small opening, or pinhole, limiting the entrance of the most diffused rays reflected by the retina in the detector. The main characteristic of such ophthalmoscope is its high optical sectioning capacity, which allows high-resolution and high-contrast topographical reconstructions. Its drawback is the need to use laser illumination and the need to perform a point-to-point scan of the entire area of the retina to be observed until obtaining the final three-dimensional image. While laser illumination gives images a monochromatic appearance, differing greatly from the chromaticity of images obtained with the retinal fundus camera, the need to scan demarcates the number of pixels of the final image and requires a certain amount of time until the entire region of interest is scanned. Furthermore, the field of view (area) that can be recorded is smaller than that which can be recorded with a fundus camera as resolution is higher.

The optical coherence tomography (OCT) scanner proposed by Huang et al. in 1991 in their article "Optical coherence tomography," Science 254, 1178-1181, uses an interferometric technique to obtain tomography images of biological tissues with high resolution, particularly high axial resolution. This instrument is based on a point-to-point scan concept similar to that of the confocal ophthalmoscope, however here the image is obtained by means of optical sectioning from low-coherence interferences between the light reflected by the different layers of retinal tissue and the light with which said tissue is illuminated. The image is then generated from successive axial measurements by performing a transverse scan, and it is provided as a two-dimensional topographical image. While axial resolution is better than that of the ophthalmoscope, transverse resolution is worse. Like in the previous case, the main advantage of OCT is its optical sectioning capacity, which allows it to obtain high-resolution 3D tomographic retinal maps. Its drawbacks are its high cost, image monochromaticity and the limited number of pixels.

Brief Description of the Invention

This invention describes a fundus camera that is capable of generating three-dimensional color images of the posterior pole of the eye. Although the fundus comprises the retina, the macula and the optic nerve, this area of the eyeball will be referred to hereinafter using the terms posterior pole, fundus or retina.

To generate 3D images of the fundus, a microlens array must be inserted between the retina and the sensor of the camera that is used to photograph it, thereby forming an integral imaging system. This imaging technique allows obtaining three-dimensional photographs of the posterior pole with polychromatic light, high resolution and complete parallax. These images can be projected from an integral imaging monitor or be used to calculate a topographical map of the fundus with high segmenting capacity. The invention advantageously combines the features of a conventional fundus camera and of the integral imaging technique for generating 3D images used to solve the main drawback of stereoscopic fundus cameras and ophthalmoscopes: visual fatigue due to the vergence-accommodation conflict.

The present invention uses integral imaging technology for generating the three-dimensional images of the fundus. The integral imaging technique is based on recording an array of basic 2D images of a 3D scene by means of a microlens array. Each of these basic images picks up a different view of the 3D scene, and as many views (basic images) as there are microlenses in the array are recorded. One of the advantages of the integral imaging technique compared to the stereoscopy technique is precisely the number of views that are recorded. In the case of integral imaging, there are as many horizontal and vertical views as there are microlenses in the array in each of these directions, whereas in stereoscopy only two horizontal views are picked up, trying to simulate the view that is perceived with respective eyes in binocular vision.

The basic images, jointly referred to as integral photograph, store discrete information of the ray map (also called «light field») emitted by the 3D scene. A topographical reconstruction of the fundus or of any element forming part of it can be made from the ray map. By means of correctly processing the ray map it is also possible to obtain a two-dimensional image of a 3D scene where planes of the scene that appear out of focus can be zoomed in on, and synthetic apertures can also be established on the captured image in order to center on the region of interest. This is known as the plenoptic technique, and the generated two-dimensional image is known as a plenoptic photograph. It is also possible to prepare an integral image for multi-view projection, with complete parallax, in an integral imaging monitor. This projected multi-view image is autostereoscopic, has depth and, unlike the projection of stereoscopic pairs, parallax, and special 3D viewing glasses are not required for viewing it.

The present invention is very useful for the diagnosis of retinal disturbances, such as glaucoma, diabetic retinopathy, macular degeneration, and any other pathology that can be diagnosed based on examination of the posterior pole, especially those that entail fundus surface topography disturbances.

Description of the Drawings

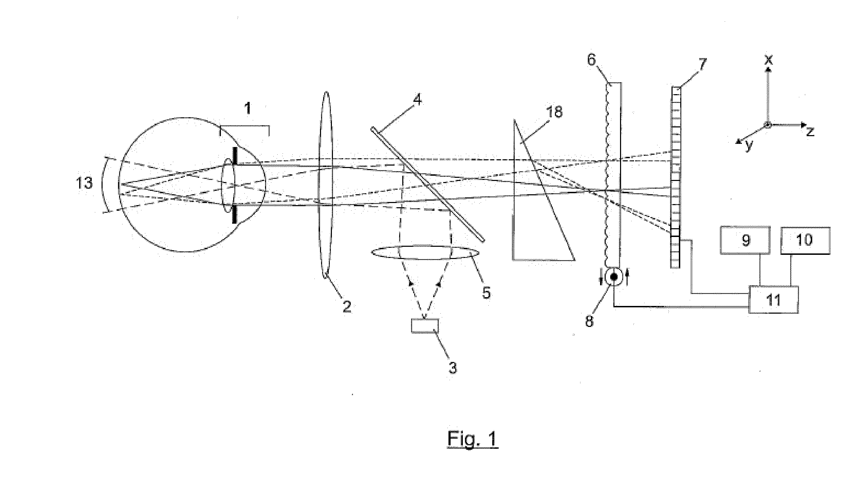

Figure 1 shows a simplified diagram of the invention with the most important components. An ophthalmoscopic lens (2) (as an optical equivalent), an illumination mechanism (3,5,4), a microlens array (6) and an array sensor (7) connected with a processing unit (11) for image processing and with image projection means, including a conventional monitor (9) and an integral imaging monitor (10), are depicted. Other optional elements for increasing resolution, namely, a phase modulator (18) and a displacement element (8), are depicted.

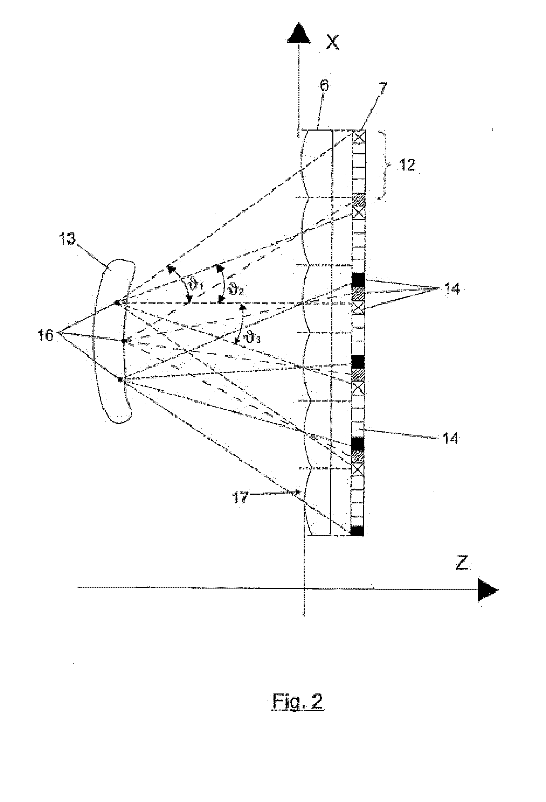

Figure 2 shows how the microlens array performs sampling. Each of the rays reflected in different directions from any illuminated point (16) of the fundus (13) goes through its corresponding microlens (17) of the array (6). Therefore a certain pixel (14) picks up the intensity of the ray that is suitably tilted.

Detailed Description of the Invention

An embodiment of the invention which must not be considered with a limiting character is described below in reference to the drawings.

Applying the concept of integral imaging to the retinal photograph allows recording a large number of views of the fundus (13) in a single photograph (integral photograph).

As seen in Figure 1, a light source (3) is projected on the fundus (13) through an optical system (5), (4) and (2) which also considers that of the eye (1). The light reflected by the fundus passes through the microlenses forming the array (6), striking the sensor (7).

An electromechanical displacement device (8), for example a piezoelelectric device, which transversely displaces the microlens array (6), thus increasing the number of effective microlenses, can be used to increase resolution of the integral imaging system.

An electronically tunable linear phase modulator (18), also known as phase wedge or electro-optical deflector, (e.g., a liquid crystal display) or an acousto-optical phase modulator, placed along the optical axis before the microlenses can be used as an alternative to this electromechanical device (8), or together with said device, deviating the rays reflected by the retina by a certain angle. With the change in direction of the path of the rays, the effect on the resolution of the integral image is equivalent to that which can be achieved with the transverse displacement of the microlenses.

Once the integral image captured by the array sensor (7) is recorded, it can be processed with the aid of a processing unit (11) and the 3D image of the retina can be projected from an integral imaging monitor (10). The processing unit (11) can also make a topographical reconstruction of the fundus (13), or of any of its elements individually, which can be displayed in the form of a topographical map or in the form of sections of different planes of the 3D scene in a conventional display or monitor (9).

As discussed, a topographical reconstruction of the fundus (13) can be calculated (processed) section-by-section from the integral photograph. The resolution of the topographical sections is equal to the resolution of the integral image. The number of segmented sections is equal to the number of views of the synthetic integral photograph.

A new integral image, called synthetic integral image, which is prepared for being projected from an integral imaging monitor (10), can be calculated from the captured integral photograph. This monitor (10) provides the observer with an actual three-dimensional reconstruction of the fundus (13). The projected 3D image is autostereoscopic, with parallax and depth, so the use of additional filters for observation is unnecessary.

By means of the coordinated action of the accommodation and vergence mechanisms of the eye, the observer can focus on different depths of the 3D image when looking at the monitor. Since it is autostereoscopic, the vergence-accommodation conflict does not occur in this observation, which allows a prolonged observation of the scene without the occurrence of visual fatigue.

As is shown in Figure 1, where a diagram of the invention can be seen, the camera has at least one ophthalmoscopic lens (2) which, with the optic system (1) of the eye, provides an enlarged image of the retina on the plane of the microlens array (6).

The array sensor (7) is adjusted such that its conjugate through each of the microlenses (7), all of which are at the same focal distances from the ophthalmoscopic lens (2) and from the front part of the optic system of the eye (formed by the cornea and the aqueous humor) coincides with the plane of the pupil (1) (which forms the aperture diaphragm of the system).

A light source (3) allows illuminating the retinal region of interest (13). A beam deflector (4) and a collecting lens (5) can be used for such illumination. The collecting lens (5) together with the ophthalmoscopic lens (2) project the image of the light source (3) on the plane of the pupil to thus form a Köhler-type retinal illumination system. Such illumination regulates the light ray cone illuminating the scene, homogenously and precisely covering the front diameter of the pupil in the specific numerical aperture thereof in order to therefore take advantage of the greatest amount of light emitted by the emitting source.

This illumination system allows capturing an entire series of basic images of the fundus (13) which, as will be seen below, contain precise information about the rays of light emitted by the retina when it is suitably illuminated.

As seen in Figure 2, the integral imaging system fundamentally consisting of the microlenses (6) and the sensor (7), works by capturing a sampling of the set of rays emitted by the 3D sample (13). A certain pixel (14) therefore picks up the intensity of the ray which, entering through the center of the corresponding microlens (17), is suitably tilted so as to be recorded in the sensor in the area (12) corresponding to that microlens. The information captured by the array sensor (the integral photograph) can be represented in the space of rays. In this space, the array sensor (7) performs continuous discrete sampling of rays emitted by the sample. The greater the number of microlenses (17) and the number of pixels (14) per microlens, the better the sampling will be and the more faithful will be the reproduction of the 3D image in a later phase. It must be observed that according to what is established by the integral imaging technique, the rays entering through a microlens (17) must be confined (by means of an optical barrier) to the area (12) in order to be correctly processed.

The number of points in which the space of rays is sampled is equal to the number of useful pixels of the sensor. To perform optimal sampling, which allows both a precise reconstruction of the space of rays and a faithful reproduction of the 3D scene in a later phase, the number of microlenses should be equal (or about equal) to the number of pixels per microlens.

Given that the resolution of the system depends on the number of microlenses (17) there are per unit area in the array (6), and given that this number is limited by the processes for manufacturing such microlenses and the size each microlens can have (the limits imposed by light diffraction must be considered herein), to increase the number of effective pixels (and with it, the effective number of microlenses) the present invention proposes adding a step-by-step transverse displacement device (8) for the step-by-step transverse displacement of the microlens array (6). An integral photograph is captured with the sensor (7) for each step of this transverse scan. These displacements have a length $\ell = p/N$, where $p$ being the spacing between microlenses (distance between consecutive microlens centers) and N being the number of steps in each $x$-$y$ Cartesian direction. This allows increasing by one factor N the sampling frequency in the space of rays. This increase in resolution can also be achieved by optical means, such as with a phase wedge (18), for example.

As is shown in Figure 1, the necessary coordination between the processes of the microlens for scanning, capturing and recording the integral photographs, processing same for constructing the synthetic integral photograph (which is not strictly necessary), and finally recording the synthetic integral photograph occurs by means of the CPU (11).

Either the original or the synthetic integral photograph can be used for three purposes:

a) Topographical reconstruction of the fundus. As multiple views are offered, and by using conventional topographical reconstruction algorithms, it is possible to generate a CAD-type topographical map of the fundus, for example, which can be seen on a conventional display. Since it contains multi-view information, this map can be turned to see the view of interest of the element of the fundus that is to be more carefully examined.

b) Topographical reconstruction of the fundus section-by-section. Optical sectioning of a 2D photograph obtained by processing the integral photograph can be performed using the plenoptic function. The resolution of the topographical sections is determined by the number of active microlenses. The number of planes into which the 3D image can be segmented is equal to the number of pixels per basic image. As a result, the use of the scanning technique in the microlenses allows increasing by one factor $\sqrt{N}$ both the resolution and the segmenting capacity of the retinal topography.

c) Projection of 3D images on an integral imaging monitor. By means of using the SPOC algorithm, it is possible to calculate a new synthetic integral photograph from the integral photograph with the set of basic images prepared to be projected on an integral imaging monitor. Given that the operation of the integral image projection system is based on the principle of the reversibility of light, and given that the configuration of the capture system does not have to be the same as the projection system, for example the size of the microlenses used for the capture are smaller than those used in projection, or the microlenses do not have the same geometry, or the distance between the sensor and the microlenses during capture is not the same as that between the monitor and the microlens array, this algorithm solves the structural differences between the capture system and the projection system, offering in this case three-dimensional images that can be directly viewed once projected without the need to use special glasses.

Specifically, this pixel sampling algorithm allows synthetically selecting the parameters of the microlens array (7), such as the focal distance, the size, the distance between microlenses (17), as well as the position thereof with respect to the monitor and the size of the reconstructed images, even the geometry of the microlenses (circular, hexagonal, square...), simulating that it has been captured in the same way that it is going to be projected. Therefore, if the projection system is not the same as the capture system, the integral photograph is adjusted so that it looks like it was recorded with a system having the same characteristics as the system used for projecting it.

The algorithm is the result of applying three processes in series: simulation of the monitor, virtual capture and homogenous scaling.

First, simulation of the monitor: the array of basic images captured with the microlenses is used as input for the algorithm.

In the second step, virtual capture: the image is transformed to simulate having been captured by means of an array of pinholes. The position of this array, the spatial period between its elements, the distance between the array and the sensor, and the number of pixels are assigned arbitrarily so that they coincide with the characteristics of the monitor that will be used as the projector.

Finally, homogenous scaling: the size of the basic synthetic images is adapted to the characteristics of the integral imaging monitor.

The number of effective pixels of the synthetic integral photograph obtained after scanning by the microlenses should be, though does not necessarily have to be, of the same order as the number of pixels of the monitor. Applying SPOC will allow obtaining an integral image in which the number of basic images must be considerably greater than the number of pixels per basic image. While the number of basic images determines the resolution of the monitor, the number of pixels per basic image determines the number of views. Between 12 and 16 views is considered a sufficient number of views, and the resolution of the monitor should be much greater.

## Claims

1. A multi-view fundus camera comprising:

- an ophthalmoscopic lens (2) configured for focusing on a plane of interest of the fundus (13) when it is illuminated with a light source (3);
- an array sensor (7) comprising a 2D pixel array in which the pixels are configured for recording the intensity and the frequency of the light reflected by the fundus (13), with said array sensor (7) coupled with image processing means (11) configured to treating a plurality of basic images formed in the pixels (14) of said array sensor (7); **characterized in that** it further comprises:
- a microlens array (6), the microlenses (17) of which are arranged forming a plane transverse to the optical axis and located between the ophthalmoscopic lens (2) and the array sensor (7), parallel to both (2,7), with each microlens (17) configured to form a basic image with a view of the fundus on a set of pixels (14) of the array sensor (7) associated with said microlens (17); where the ophthalmoscopic lens (2) and the microlens array (6) are arranged such that the image of an arbitrary plane of the fundus (13) is formed on the microlens array (6), and said microlens array (6) is in turn arranged, in conjunction with the external optic system of the eye of the patient and the ophthalmoscopic lens (2), to form an image of the pupil of the eye on the array sensor (7).

**2.** The camera according to claim 1, **characterized in that** it further comprises displacement means (8) configured to transversely displace the optical axis of the microlens array (6) in coordination with the image processing means (11) to acquire a set of basic images formed in the pixels (14) in each transverse displacement.

**3.** The camera according to claim 2, **characterized in that** the displacement means (8) comprise a piezoelectric mechanism.

**4.** The camera according to any one of the preceding claims, **characterized in that** it further comprises a linear phase modulator (18) coupled to the microlens array (6) the phase-modulating characteristics of which can be selected from electro-optical and acoustic-optical.

**5.** The camera according to any one of the preceding claims, **characterized in that** the light source (3) comprises a beam deflector (4) and a collecting lens (5), such that the collecting lens (5) together with the ophthalmoscopic lens (2) project the image of the light source (3) onto the plane (1) of the pupil of the eye (13).

**6.** The camera according to any one of the preceding claims, **characterized in that** the number of microlenses (17) of the array (6) is substantially equal to the number of pixels (14) per microlens (17).

**7.** The camera according to any one of the preceding claims, **characterized in that** the geometry of the microlenses (17) is selected from: circular, hexagonal or square.

**8.** The camera according to any one of the preceding claims, **characterized in that** the processing means (11) are configured to reconstruct a topographical map of the fundus (13) from the information corresponding to a plurality of views picked up by the microlens array (7).

**9.** The camera according to claim 8, **characterized in that** it further comprises image projection means (10, 11).

**10.** The camera according to any one of the preceding claims 1 to 9, **characterized in that** the light source (3) emits in the visible radiation range, between 380 nm and 780 nm.

**11.** The camera according to any one of the preceding claims 1 to 9, **characterized in that** the light source (3) emits infrared radiation comprised between 780 nm and 3,000 nm.

Fig. 1

Fig. 2

## INTERNATIONAL SEARCH REPORT

| International application No. |
| --- |
| PCT/ES2012/070726 |

**A. CLASSIFICATION OF SUBJECT MATTER**

*A61B3/12* (2006.01)

According to International Patent Classification (IPC) or to both national classification and IPC

**B. FIELDS SEARCHED**

Minimum documentation searched (classification system followed by classification symbols)
A61B, G02B, G03B

Documentation searched other than minimum documentation to the extent that such documents are included in the fields searched

Electronic data base consulted during the international search (name of data base and, where practicable, search terms used)

EPODOC, INVENES, WPI, INSPEC

**C. DOCUMENTS CONSIDERED TO BE RELEVANT**

| Category* | Citation of document, with indication, where appropriate, of the relevant passages | Relevant to claim No. |
| --- | --- | --- |
| A | US 7290880 B1 (YARON, A. ET AL.) 06.11.2007, abstract; column 1, lines 15-41; column 3, line 61 - column 4, line 15; column 4, line 58 - column 7, line 16; column 8, line 6 - column 9, line 49; figures 1 - 3b. | 1, 5, 8-11 |
| A | US 2003/0038921 A1 (NEAL, D. ET AL.) 27.02.2003, abstract; paragraphs [0022]-[0030], [0044]-[0055]; figures 1-3. | 1, 5, 8-11 |
| A | US 5995759 A (KOHAYAKAWA, Y.) 30.11.1999, the whole document. | 1, 5, 8-11 |
| A | JP 2006087793 A (KOWA CO.) 06.04.2006, the whole the document. | 1, 5, 8, 9 |

☒ Further documents are listed in the continuation of Box C.     ☒ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance. | |
| "E" earlier document but published on or after the international filing date | |
| "L" document which may throw doubts on priority claim(s) or which is cited to establish the publication date of another citation or other special reason (as specified) | "X" document of particular relevance; the claimed invention cannot be considered novel or cannot be considered to involve an inventive step when the document is taken alone |
| "O" document referring to an oral disclosure use, exhibition, or other means. | "Y" document of particular relevance; the claimed invention cannot be considered to involve an inventive step when the document is combined with one or more other documents , such combination being obvious to a person skilled in the art |
| "P" document published prior to the international filing date but later than the priority date claimed | |
| | "&" document member of the same patent family |

| Date of the actual completion of the international search | Date of mailing of the international search report |
| --- | --- |
| 07/02/2013 | **11/02/2013)** |

| Name and mailing address of the ISA/ | Authorized officer |
| --- | --- |
| OFICINA ESPAÑOLA DE PATENTES Y MARCAS Paseo de la Castellana, 75 - 28071 Madrid (España) Facsimile No.: 91 349 53 04 | Ó. González Peñalba Telephone No. 91 3495475 |

Form PCT/ISA/210 (second sheet) (July 2009)

# INTERNATIONAL SEARCH REPORT

International application No.

PCT/ES2012/070726

C (continuation). DOCUMENTS CONSIDERED TO BE RELEVANT

| Category * | Citation of documents, with indication, where appropriate, of the relevant passages | Relevant to claim No. |
|---|---|---|
| A | MARTÍNEZ-CUENCA, R. et al.: "Progresses in 3D maging and display by integral imaging". Three-Dimensional Imaging, Visualization and Display 2009, Proc. of SPIE, 2009, Vol. 7329, págs. 1-7. | - |
| A | NAVARRO, H. et al.: "Optical slicing of large scenes by synthetic aperture integral imaging". Three-Dimensional Imaging, Visualization and Display 2010. Proc. of SPIE, 2010, Vol. 7690, págs. 1-7. | - |

Form PCT/ISA/210 (continuation of second sheet) (July 2009)

## INTERNATIONAL SEARCH REPORT

Information on patent family members

| International application No. |
| --- |
| PCT/ES2012/070726 |

| Patent document cited in the search report | Publication date | Patent family member(s) | Publication date |
| --- | --- | --- | --- |
| US7290880 B1 | 06.11.2007 | NONE | |
| US2003038921 A1 | 27.02.2003 | US6634750 B2 | 21.10.2003 |
| | | WO02075367 A2 | 26.09.2002 |
| | | JP2005506107 A | 03.03.2005 |
| | | JP4199000B2 B2 | 17.12.2008 |
| | | EP2008576 A2 | 31.12.2008 |
| | | EP1367935 A2 | 10.12.2003 |
| | | AU2002305045 A1 | 03.10.2002 |
| | | AT508676T T | 15.05.2011 |
| | | AT408368T T | 15.10.2008 |
| US5995759 A | 30.11.1999 | JP10165372 A | 23.06.1998 |
| JP2006087793 A | 06.04.2006 | US2006082726 A1 | 20.04.2006 |
| | | US7287854 B2 | 30.10.2007 |
| | | JP4585824B2 B2 | 24.11.2010 |

Form PCT/ISA/210 (patent family annex) (July 2009)

**REFERENCES CITED IN THE DESCRIPTION**

**Patent documents cited in the description**

- US 7798642 B2
- US 7290880 B1

**Non-patent literature cited in the description**

- **H. NAVARRO ; R. MARTÍNEZ-CUENCA ; G. SAAVEDRA ; M. MARTÍNEZ-CORRAL ; B. JAVIDI.** 3D integral imaging display by smart pseudoscopic-to-orthoscopic conversion. *Opt. Express,* 2010, vol. 25, 25573-25583
- **WEBB et al.** Confocal scanning laser ophthalmoscope. *Appl. Opt.,* 1987, vol. 26, 1492-1499
- **HUANG et al.** Optical coherence tomography. *Science,* 1991, vol. 254, 1178-1181